# EUROPEAN PATENT APPLICATION

(11) **EP 0 988 834 A2**
(43) Date of publication of application: **29.03.2000**
(21) Application number: 99203118.7
(22) Date of filing: 23.09.1999
(51) Int. Cl.: A61C 5/02

(54) **Devices for use in disinfection treatments and method for cleansing dental implantological elements**

(30) Priority: 23.09.1998 NL 1010160
(71) Applicant: Schafrat, B., 1019 EE Amsterdam (NL)
(72) Inventor: Schafrat, B., 1019 EE Amsterdam (NL)
(74) Representative: de Vries, Johannes Hendrik Fokke

(57) **Abstract**

The invention relates to devices for use in disinfection treatments, for example in parodontology. The invention relates in particular to a needle for use in the subgingival cleansing of a mouth cavity. The needle is to that end provided with a central, axial passage for supplying ozone and with means for connection to a source of ozone. Such a needle enables an improved cleansing of the subgingival pocket. The invention furthermore relates to a method for cleansing prostheses. To that end, the prostheses are contacted with ozone for some time.

## Description

The invention relates to devices for use in disinfection treatments. More in particular, the invention relates to such means for use in the disinfection treatment of a mouth cavity.

Disinfective cleansing both of parts of the human body and of objects is known per se. Generally, the object or the part of the body to be disinfected is brought into contact with a disinfectant.

In this connection it is known to cleanse a mouth cavity by a disinfection treatment. With the known methods, a disinfective fluid or gel, such as a solution of chlorhexidine, of hydrogen peroxide or of sodium perborate, is introduced into the mouth cavity. Said liquids are generally applied as a rinsing fluid or as a gel, by means of which it is attempted to disinfect the niche in question completely, but in practice not all places are adequately disinfected. In particular adequate subgingival disinfection of the pathologic pocket is difficult. A longer rinsing time is not a suitable solution, because it increases the risk of the following drawbacks occurring: allergic reactions and damage to tissue that is essential for recovery.

Furthermore it is known to fight athlete's foot, for example, with a disinfection treatment. To that end, means in the form of an ointment are locally applied, or medicine must be taken orally. Due to the difficulty in reaching in particular the fungi etc. that are present under the nails, treatment is hardly ever complete.

The object of the invention is to provide devices for use in disinfection treatments, wherein a disinfectant can be used which does not exhibit drawbacks such as the above.

In order to accomplish that objective, the invention is characterized in that the device mentioned in the introduction is a needle which is provided with a central, axial passage for supplying ozone to a body to be disinfected, and with means for connection to a source of ozone. The term body as used herein is understood to include both parts of the human body and parts of an animal body as objects.

It is a well-known fact in the industry that ozone has disinfecting properties, that is, it kills microorganisms, spores and viruses. A major drawback of the use of ozone is its toxicity upon being inhaled. Because of this toxicity, it has not appeared to be possible so far to use ozone for in vivo applications in dentistry or parodontology. The advantage of the use of ozone in comparison with the known disinfectants is that there is no risk of resistant bacterial strains developing.

The invention makes it possible to use ozone in subgingival treatments. The needle may be made of any suitable, ozone-resistant material.

The invention furthermore relates to a suction device which can be used for removing gaseous ozone or other harmful gases that may been released from the mouth cavity. Such a suction device comprises an annular structure, which is placed in the mouth cavity, on the inner side of the lips, without making contact with the dental elements to any significant degree.

In order to be able to ascertain the amount of ozone that is supplied during a treatment, the invention furthermore provides a method for determining an amount of ozone, possibly mixed with another gas, which is supplied during a treatment with ozone. To this end, a known portion of the ozone-containing gas is passed through a potassium iodide solution, which will result in discolouration of the solution as a function of the amount of ozone that has been passed through the solution.

Finally, the invention provides devices for the disinfective cleansing of the other mouth mucosa and skin surfaces in general.

The invention will be now explained in more detail with reference to the drawings, which show preferred embodiments of the invention. In the drawings:
Figures 1 and 1A are perspective views of a needle according to the invention;
Figure 2 is a perspective view of a suction device according to the invention, which is positioned in a mouth cavity;
Figure 3 is a sectional view of the suction device of Figure 2, along line III-III;
Figures 4 and 5 show a holder for use in the measuring method according to another aspect of the invention; and
Figures 6 - 9 show means for cleansing other mucous membrane of the mouth and skin surfaces in general.

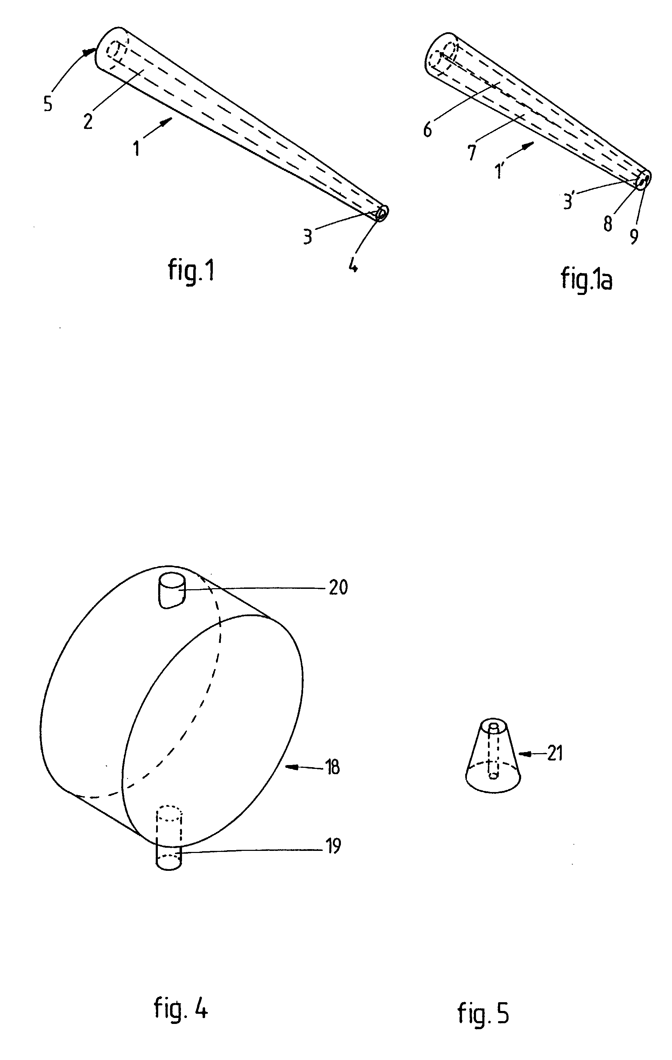

Figure 1 shows a needle which can be suitably used in the disinfective cleansing of the mouth cavity, in particular a subgingival pocket or other niche in a body, for example under a nail. The needle 1 is to that end provided with a central, axial passage 2 for passing ozone therethrough. The end 3, which is provided with an outlet opening 4, can be inserted into a niche, for example subgingival pocket, for that purpose. Ozone can be supplied to the pocket by connecting the other end 5 of the needle, via suitable means, to an ozone source (not shown) in a manner which is known per se in the industry.

In this manner the needle 1 according to the invention, when used in a subgingival treatment, makes it possible to achieve a very good disinfection of the location to be treated. Since the ozone can be directly introduced into the subgingival pocket with the needle 1 according to the invention, a very good contact of ozone with the microorganisms present in the pocket is obtained. As a result of this, the amount of gaseous ozone which finds its way into the mouth cavity is very small. Preferably, needle 1 is a subgingival application needle, and the dimension and/or the shape of needle 1, which preferably terminates in a sharp point, is adapted to the clinical situation.

Ozone combines very well with aqueous liquids. When contacted with water, a redox reaction develops almost immediately. The disinfective effect of ozone is retained thereby. In addition, the transport possibility and the transport rate of ozone are strongly enhanced.

Due to the good transport possibility of ozone, it is also very well possible to combine ozone irrigation of a pocket with irrigation thereof with an aqueous solution. Preferably, a physiological saline solution (PBS) is used for this purpose, so that the biocompatibility of the applied mixture will be very high.

Figure 1A shows a preferred embodiment of a needle 1' according to the invention, which can suitably be used for simultaneously applying ozone and a liquid. In Figure 1, needle 1' is provided with two separate axial passages 6, 7, which are suitable for passing ozone and a liquid, respectively. The ozone and the liquid are supplied to a pocket via outlets 8, 9 at the end 3'. It is also possible to combine the ozone with the liquid before the ozone exits needle 1'. In that case there will be only one outlet.

Generally, liquids are moreover ideal mediums for transmitting ultrasonic vibrations, and consequently they are suitable for use as a conducting medium for removing materials of a varying nature that have adhered to bodies which are present in the liquid from said bodies by means of ultrasonic vibrations.

According to the invention it has become apparent that the application of ozone to a liquid, combined with ultrasonic vibrations, produces an unexpected synergetic effect. Accordingly, the invention also relates to needles which are suitable for applying both ozone and a liquid to a pocket, and wherein the needle is suitable for transmitting ultrasonic vibrations. In that case, the needle includes means for connection to a cavitron device, for example.

In the case of implants, the base of which is often made of a relatively soft material, it is preferable to use needles 1, 1' which are made of a material softer than that of the implants, that is, a material whose hardness is lower than that of the material from which the implant is made. The relatively low hardness is selected with a view to treating titanium pillars, for example, in which case there is no risk of the titanium surfaces being damaged whilst achieving an optimum cleansing and sterilisation result. The material may be any suitable, relatively soft material, such as a tin-containing alloy.

Furthermore it is possible to manufacture flexible needles from relatively soft materials. This makes it possible to reach the subgingival pockets which are difficult to get to by adapting the shape of the needle as required. These needles may be suitable for use only once.

Besides the possible combinations of ozone and liquid rinsing agents, such as a physiological saline solution, for example, gaseous ozone also has a cleansing effect of its own. Unexpectedly, it has become apparent that ozone provides an effervescent effect, in particular in protein-rich suspensions. This results in the discharge of the treated fluid, especially when gassing a closed space provided with a single opening.

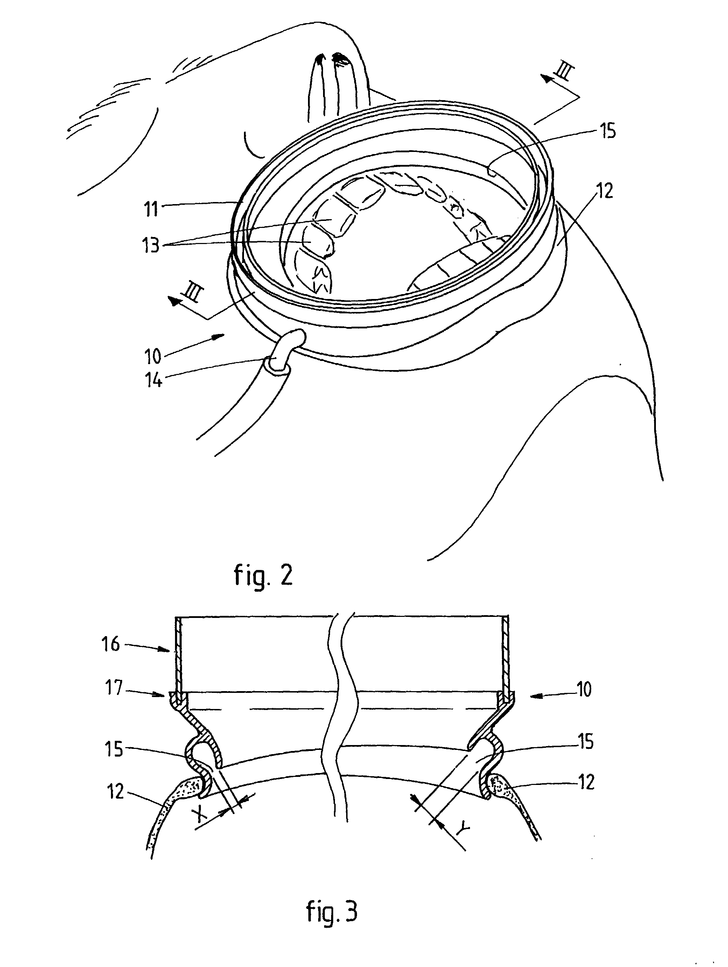

In order to remove the ozone that is released in the mouth cavity, the invention provides a suction device for suctioning harmful substances from a mouth cavity. Figure 2 shows such a suction device 10, which is present in a mouth cavity. As is shown in Figures 2 and 3, the suction device 10 has a substantially annular structure 11, which is placed in the mouth cavity, on the inside of lips 12, without making contact with dental elements 13 to any significant degree, wherein the suction device 10 includes means 14 to be connected to a suction plant (not shown), and wherein the suction device 10 is circumferentially provided, on the side facing the mouth cavity, with a slotted suction opening 15 for suctioning gas therethrough, wherein the suction opening 15 is so configured that the volume flow of the suctioned gas is substantially uniform over the entire circumference. For example, the dimension of the opening near means 14 is as indicated at x in Figure 3, and the dimension on the opposite side of the annular structure is as indicated at y.

The suction device 10 according to the invention preferably includes connecting means 14 for connection to a device by means of which the ozone can be discharged into the atmosphere, for example, or by means of which the ozone can be decomposed into oxygen, for example, or a device which makes it possible to recover the unused ozone for the purpose of using it again.

When a patient undergoes a treatment wherein ozone is used, the patient will generally lie with his head inclined backwards. When the mouth is wide open (also when this happens passively), the throat opening will automatically close, so that breathing will take place through the nose. In order to prevent ozone that may be released from the mouth cavity from being inhaled via the nose, the suction device 10 according to the invention is preferably provided with screening means 16. Said screening means 16 may simply consist of a flexible, strip-shaped, preferably transparent plate, which can be fitted by means of suitable fitting means 17, which may be present on said suction device 10.

The suction device 10 cannot only be used quite well for suctioning ozone during a treatment whereby ozone is applied. The suction device 10 can also be used in dental treatments wherein gaseous mercury (Hg) may be released. The danger of inhaling toxic mercury vapours is thus reduced both for the patient and for the person carrying out the treatment.

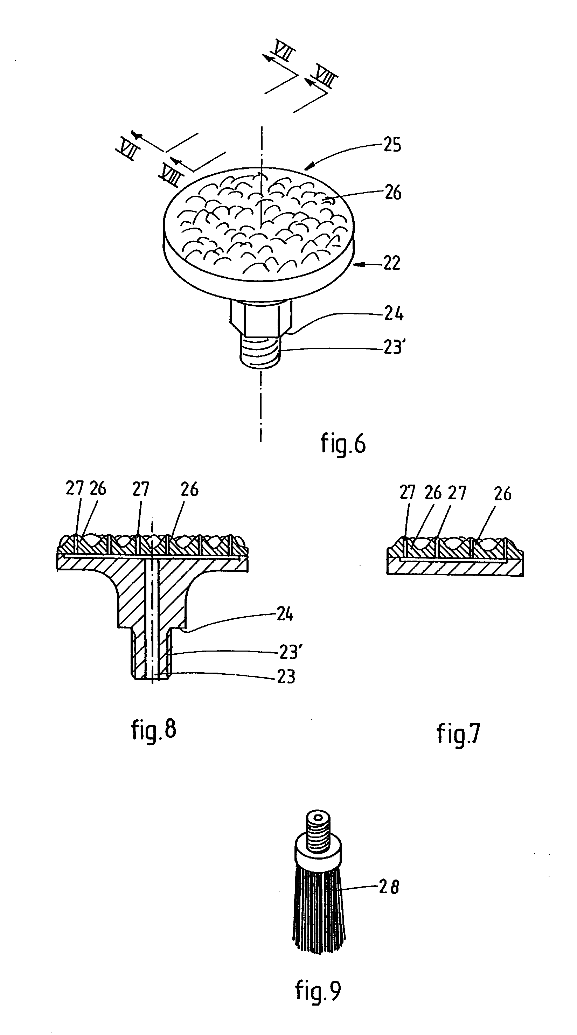

The invention furthermore relates to a method for determining the amount of ozone which has been administered to a patient during a treatment. Figure 4 shows a holder 18 which contains an amount of KI solution and which includes closable openings 19, 20, which enable the supply and discharge of ozone-containing gas. Opening 19 for supplying gas is provided at the bottom side of holder 18, as a result of which the ozone-containing gas is passed through the solution. Opening 20 for discharging unreacted gas is provided at the upper side of the holder, at a place such that no liquid can flow out of holder 18. Openings 18, 19 may be configured in any desirable manner. Figure 5 shows an example of a connecting means for connection to opening 19, which is provided with a rubber closure at one end 21 and which can be pierced with a needle which may be identical to the needles according to the invention.

It is preferable that part of the gas flow which is supplied to a patient or to a cleansing treatment, and which contains the ozone, is diverted to the potassium iodide solution, so that a discolouration of the solution is obtained. The degree of discolouration depends on the amount of ozone that has been added to the solution. The colour of the solution can then be compared with a colour set. When the amount of solution present in holder 18, the concentration of the KI therein, and also the level of the liquid in the holder are known, the amount of ozone which is passed through the holder can simply be determined on the basis of the degree of discolouration by indicating the corresponding amount of ozone at every colour of the colour set. When the ratio of the subflow to the main flow supplied to the treatment is known, the total amount of ozone that has been administered to the patient can be determined in a simple manner. When said ratio is constant, the amount of ozone that has been administered to the patient can be indicated on the colour set. Thus, a method is provided by which the amount of ozone can be determined in a simple manner. In addition, this method does not influence the administration of ozone to the patient.

In order to provide a further disinfection treatment of the mouth cavity, the invention also relates to means for cleansing the oral mucosal surfaces, that is, both the buccal mucosa and the other mouth mucosa, and the tongue. It can also be used for skin surfaces outside the mouth. As is shown in Figures 6 - 9, such a device is made up of a substantially cylindrical body 22. Said body 22 includes means 23 for connection to an ozone source, and possibly for connection to a liquid source. Device 22 is provided on one end surface 24 thereof with means 23' for connection to a cavitron device for transmitting ultrasonic vibrations to the body, and on the other end surface 25 it is provided with a multitude of protuberances 26 as well as with openings 27 for supplying ozone, possibly combined with a liquid. Figure 7 is a sectional view along line VII-VII in Figure 6, showing protuberances 26 and openings 27 for the passage of ozone, for example. Figure 8 is a sectional view along line VIII-VIII in Figure 6, wherein also the connection means 23 and 23' are shown.

If the mucous membrane to be treated should require this, the device may comprise a multitude of flexible, wire-like elements 28 rather than protuberances 26. Said elements may exhibit a stiffness that can be varied as desired. Such a device is shown in Figure 9.

The invention furthermore relates to an ozone set which can be used in the disinfection of a mouth cavity. Said set comprises the above-described needle 1, 1' as shown in Figures 1 and 1A, and preferably also the above-described suction device 10 as shown in Figures 2 and 3. The set may also comprise means for cleansing the buccal mucosa, as well as the holder for use in the method for determining the amount of ozone which has been supplied during a treatment.

The needle according to the invention is also suitable for cleansing implantological elements. The term implantological elements is understood to include implants as well as abutments and suprastructures. Accordingly, the invention also relates to a method for cleansing an implantological element with a needle according to the invention, wherein the prosthesis, prior to being introduced into the jaw or teeth, is contacted with ozone for a period of time which is sufficiently long for obtaining disinfection. The amount of time that is required will depend on the amount of ozone that is supplied per unit time. It may for example depend on the needle that is being used or on the concentration of ozone in the gas that is being supplied. According to one preferred embodiment, an implantological element is cleansed in a liquid bath by a combination of ozone and ultrasonic vibrations. Accordingly, the invention also provides an ultrasonic cleansing bath, comprising ozone as a disinfectant.

According to the invention, means for use in the disinfective cleansing of the mouth cavity are therefore provided. Thus, complete disinfection has become possible. Since said means make it possible to carry out said disinfection with ozone, there is no risk of the microorganisms becoming resistant in case disinfection should be incomplete. The invention is not restricted to the above-described embodiment as shown in the figures. For example, a needle according to the invention may be curved so as to be capable of reaching the subgingival pocket in the entire mouth cavity. The needle may furthermore be slightly flexible. Furthermore it is possible to use the needles as described above in the treatment of microbiological skin disorders, such as athlete's foot. To that end, the needle can simply be inserted under the nail, so that administration of ozone, possibly combined with a liquid, can take place. As a result of this, an excellent disinfection of the treated location is obtained.

## Claims

1. A device for use in a disinfection treatment, **characterized in that** said device is a needle which is provided with a central, axial passage for supplying ozone to a body to be disinfected, and with means for connection to a source of ozone.

2. A device according to claim 1, wherein said device is suitable for use in the disinfective cleansing of a mouth cavity, for example a subgingival pocket.

3. A device according to claim 2, wherein said device is also suitable for supplying a liquid solution to the subgingival pocket and wherein said device comprises means for connection to a liquid source.

4. A device according to any one of the claims 1 - 3, wherein said device comprises means for connection to a source of ultrasonic vibrations.

5. A device according to any one of the claims 1 - 4, wherein said device is made of a material having a hardness lower than that of an implant in a jaw, for example of a tin-containing alloy.

6. A suction device for suctioning harmful substances from a mouth cavity, comprising an annular structure, which is placed in the mouth cavity, on the inner side of the lips, without making contact with the dental elements to any significant degree, wherein the suction device includes means to be connected to a suction plant, and wherein the suction device is circumferentially provided, on the side facing the mouth cavity, with a slotted suction opening for suctioning gas therethrough, and wherein the suction opening is so configured that the volume flow of the suctioned gas is substantially uniform over the entire circumference.

7. A suction device according to claim 6, comprising screening means for screening the nose from harmful substances exiting from the mouth, said screening means comprising a flexible, strip-shaped plate, which is provided with means for being attached to said suction device.

8. A device for use in the disinfective cleansing of skin surfaces and mucosal surfaces, **characterized in that** said device is made up of a substantially cylindrical body including means for connection to an ozone source, and possibly for connection to a liquid source, and which is provided on one end surface thereof with means for connection to a cavitron device for transmitting ultrasonic vibrations to the body, and which is provided on the other end surface with a multitude of protuberances as well as with openings for supplying ozone, possibly combined with a liquid.

9. A device for use in the disinfective cleansing of skin surfaces and mucosal surfaces, **characterized in that** said device is made up of a substantially cylindrical body including means for connection to an ozone source, and possibly for connection to a liquid source, and which is provided on one end surface with means for connection to a cavitron device for transmitting ultrasonic vibrations to the body, and which is provided on the other end surface with a multitude of flexible, wire-like elements as well as with openings for supplying ozone, possibly combined with a liquid.

10. A set for use in the purification of ozone, comprising means according to any one of the claims 1 - 5 with dimensions and through-flow capacities adapted to various clinical situations.

11. A set according to claim 9, further comprising suction means according to any one of the claims 6 or 7.

12. A set according to claim 10 or 11, further comprising means according to claim 8.

13. A set according to any one of the claims 10 - 12, further comprising means according to claim 9.

14. A method for the disinfective cleansing of an implantological element, **characterized in that** said implantological element is contacted with ozone for a period of time which is sufficiently long for obtaining disinfection.

15. A method according to claim 14, wherein said implantological element is placed into a liquid bath to which ozone is supplied, preferably in combination with ultrasonic vibrations.

16. An ultrasonic cleansing bath, comprising a liquid, for example water, and ozone as a disinfectant.

17. A method for determining an amount of ozone, possibly mixed with another gas, which is supplied during a treatment with ozone, wherein a known portion of the ozone-containing gas is passed through a potassium iodide solution, as a result of which a discolouration of the solution is obtained as a function of the amount of ozone that has been passed through the solution, and wherein said discolouration is compared with a colour set for the purpose of determining the total amount of ozone that has been supplied during the treatment.

18. A device according to claim 1, wherein said device is suitable for use in the disinfective treatment of athlete's foot.
